# EUROPEAN PATENT APPLICATION

(11) **EP 3 805 397 A1**
(43) Date of publication of application: **14.04.2021**
(21) Application number: 19810697.3
(22) Date of filing: 31.05.2019
(51) Int. Cl.: C12N 15/867, A61K 48/00, A61P 25/16, A61P 25/28

(54) **GENE SEQUENCE CONSTRUCT USED FOR TREATMENT OF CENTRAL NERVOUS SYSTEM DISEASES**

(30) Priority: 31.05.2018 CN 201810553745; 05.07.2018 WO PCT/CN2018/094686
(71) Applicant: Kanglin Biotechnology (Hangzhou) Co., Ltd, Hangzhou, Zhejiang 310000 (CN)
(72) Inventor: WU, Haoquan, Hangzhou, Zhejiang 310000 (CN)
(74) Representative: Mewburn Ellis LLP
(86) International application number: PCT/CN2019/089432
(87) International publication number: WO 2019/228487

(57) **Abstract**

A gene sequence construct used for the treatment of central nervous system diseases: by means of the construction of an auto-processing expression vector, tyrosine hydroxylase (TH), GTP-cyclohydrolase I (GCH1), aromatic amino acid dopa decarboxylase (AADC), and so on may be simultaneously expressed; proteins are connected by means of an auto-processing unit (APU); the use of a viral vector to introduce the construct into a target cell may ultimately result in the high-efficiency expression of tyrosine hydroxylase (TH), GTP-cyclohydrolase I (GCH1), aromatic amino acid dopa decarboxylase (AADC), and so on having independent functions, being used in the prevention or treatment of Parkinson's disease, Alzheimer's disease and other neurodegenerative diseases.

## Description

### TECHNICAL FIELD

The present invention relates to the field of gene therapy, in particular to a type of gene sequence constructs for the treatment of central nervous system diseases. The gene sequence constructs can be used to prevent or treat Parkinson's disease, Alzheimer's disease and other neurodegenerative diseases.

### TECHNICAL BACKGROUND

Parkinson's disease (PD) is a neurodegenerative disease, characterized by the loss of dopaminergic neurons in the *substantia nigra,* resulting in a decrease in midbrain dopamine levels to cause the disease. Parkinson's disease affects about 1% of the world's population over 55 years of age. As the society ages, the patient population will continue to grow. The common treatment for Parkinson's disease is oral administration of dopamine's precursor-levodopa, which can to some extent relieve symptoms. However, with disease progression, levodopa treatment is unsatisfactory.

Gene therapy has unique advantages for the treatment of Parkinson's disease. By targeted delivery of dopamine synthesis genes to the striatum, dopamine can be synthesized and released in the striatum, thus making the treatment of Parkinson's disease more effective. Tyrosine is catalyzed by tyrosine hydroxylase (TH) to synthesize levodopa, and then aromatic amino acid decarboxylase (AADC) converts levodopa to dopamine. TH needs tetrahydrobiopterin as a coenzyme, and GTP-cyclohydrolase 1 (GCH1) catalyzes the synthesis of tetrahydrobiopterin, and therefore the expression of TH, AADC and GCH1 can efficiently synthesize dopamine (Azzouz M et al., 2002. J Neurosci. 22 (23): 10302-12 .; Jarraya B et al., 2009. Sci Transl Med. 1 (2): 2).

In 2014, a lentiviral vector based on equine infectious anemia virus (EIAV) carrying two internal ribosome entry sites (IRES) linking three key dopamine synthetases showed good results in a phase I/II clinical trial (Palfi S et al., 2014. Lancet. 383 (9923): 1138-46.). However, this method has several problems. For example, IRES expression of three genes often leads to imbalanced expression. Therefore, Stewart HJ et al. replaced the IRES element with a linker peptide to produce a fusion protein containing two or more of the three enzyme activities required for dopamine synthesis (Stewart HJ et al. 2016. Hum Gene Ther Clin Dev.27 (3): 100-10.). However, TH, AADC and GCH1 are expressed as independent proteins under natural conditions, and the fusion protein may affect the efficiency of dopamine synthesis. Therefore, to find a new protein co-expression method, to select a suitable vector for delivery into the target cells, and to express efficiently in them, are still problems to be solved for the treatment of Parkinson's disease.

### SUMMARY OF THE INVENTION

The purpose of the present invention is to address the shortcomings of the existing treatment technology, by construction of auto-processing expression vectors, to express tyrosine hydroxylase (TH), GTP-cyclohydrolase I (GCH1), aromatic amino acid dopa decarboxylase (AADC), and nervous system growth factors, etc. The proteins are linked by an auto-processing unit (APU). Viral vectors are used for delivery into target cells, which can result in highly efficient expression of independently functional tyrosine hydroxylase (TH), GTP-cyclohydrolase I (GCH1) and aromatic amino acid dopa decarboxylase (AADC), etc., for the treatment of Parkinson's disease.

The present invention includes the following: a gene sequence construct for the treatment of a central nervous system disease, the construct comprising nucleotide sequences that are related to the treatment of the central nervous system disease and are linked by an auto-processing unit (APU).

Preferably, said nucleotide sequences related to the central nervous system disease in the gene sequence construct for the treatment of the central nervous system disease are selected from two or more of the nucleotide sequences of tyrosine hydroxylase (TH), GTP-cyclohydrolase I (GCH1), aromatic amino acid dopa decarboxylase (AADC), and a nervous system growth factor.

Said nucleotide sequences comprise at least two nucleotide sequences that are linked by an auto-processing unit (APU); said auto-processing unit (APU) comprising an N-terminal auto-processing domain and/or a C-terminal auto-processing domain.

Preferably, the nervous system growth factor comprises nerve growth factor (NGF), brain-derived neurotrophic factor (BDNF), neurotrophin-3 (NT-3), neurotrophin-4/5 (NT-4/5), neurotrophin-6 (NT-6), ciliary neurotrophic factor (CNTF), glial cell line-derived neurotrophic factor (GDNF) and a GDNF family molecule (a naturally occurring analog of GDNF, neurturin, persephin and artemin).

Preferably, the gene sequence construct for the treatment of the central nervous system disease comprises tyrosine hydroxylase (TH), GTP-cyclohydrolase I (GCH1), aromatic amino acid dopa decarboxylase (AADC).

Said nucleotides sequences comprise at least two nucleotide sequences that are linked by an auto-processing unit (APU).

Preferably, said N-terminal auto-processing domain comprises Intein, B-type bacterial intein-like domain (BIL), Furin sequence, or a derivative thereof.

Preferably, said C-terminal auto-processing domain comprises a 2A peptide or a 2A-like peptide.

Preferably, the 2A peptide or 2A-like peptide in the gene sequence construct for the treatment of the central nervous system disease comprises a 2A peptide derived from foot-and-mouth disease virus (F2A), a 2A peptide derived from porcine teschovirus virus (P2A), a 2A peptide derived from insect virus (T2A), or a 2A peptide derived from equine rhinitis virus (E2A).

The first described auto-processing 2A peptide was derived from foot-and-mouth disease virus (FMDV). FMDV belongs to the genus Foot-and-Mouth Disease Virus of the small RNA virus family. The high-order structure of protease 2A encoded by the FMDV genome can cause steric hindrance to the center of the ribosomal peptidyl transferase, resulting in the failure to form a normal peptide chain linkage. However, at the same time the ribosome can continue to translate downstream proteins, thereby having a proteolytic enzyme-like effect, "cleaving" the two proteins *in cis.* Similar to FMDV, heart virus in the family of *Picornaviridae,* Theiler's murine encephalomyelitis virus, equine rhinitis virus, porcine teschovirus virus, etc. also contain a 2A peptide. In addition, gene sequences with similar functions of 2A peptide have been found in insect virus, type C rotavirus and trypanosome repeat sequences. The 2A peptide or 2A-like peptide auto-processing sequence, like the internal ribosomal entry site (IRES), is often used for multi-gene expression to achieve the independent expression of two or more non-fused exogenous proteins. Compared with IRES, 2A peptides or 2A-like peptides have apparent advantages in the construction of multi-gene expression vectors. For example, 2A peptides or 2A-like peptides are relatively small, and the expression of the upstream and downstream genes linked by the 2A element is well balanced. The present invention uses auto-processed peptides P2A to link TH, AADC and GCH1 to achieve independent and efficient expression of the three proteins.

A viral vector genome, said viral vector genome comprising any of the above-described gene sequence constructs for the treatment of a central nervous system disease.

Said viral vector comprises a lentiviral vector or an adeno-associated viral vector.

A lentiviral vector system, said lentiviral vector system comprising a genome comprising an above-described gene sequence construct for the treatment of a central nervous system disease, and one or more nucleotide sequences encoding the gag and pol proteins and other nucleotide sequences of essential virus packaging components.

A biological product comprising any of the gene sequence constructs described above, and its use in the treatment and/or prevention of Parkinson's disease, Alzheimer's disease and other neurodegenerative diseases.

A biological product comprising any of the gene sequence constructs described above, comprising a viral vector construct system comprising an above gene sequence, and other necessary viral packaging components, to produce virus particles with a pharmaceutically acceptable carrier or a diluent to form a biological product, and for preparing a medicament for producing dopamine *in vivo,* and its use in the treatment and/or prevention of Parkinson's disease, Alzheimer's disease and other neurodegenerative diseases.

Specifically, a gene sequence construct, said construct comprising selection of two or more of the nucleotide sequences of tyrosine hydroxylase (TH), GTP-cyclohydrolase I (GCH1), aromatic amino acid dopa decarboxylase (AADC).

Said nucleotide sequences comprise at least two nucleotide sequences linked by an auto-processing unit (APU).

The following is a selected gene sequence construct comprising the nucleotide sequences of tyrosine hydroxylase (TH), GTP-cyclohydrolase I (GCH1), aromatic amino acid dopa decarboxylase (AADC), the nucleotide sequences comprising at least two sequences that are linked by an auto-processing unit (APU). The gene sequence construct comprises the following modes of construction:
TH-_{APU}-CH1-_{APU}-AADC; TH-_{APU}-CH1_{-other sequence-}AADC; TH_{-other sequence-}CH1-_{APU}AADC;
TH-_{APU}-AADC -_{APU-}CH1; TH_{-other sequence-}AADC_{-APU-}CH1; TH-_{APU}-AADC-_{other sequence-}CH1;
CH1_{-APU-}TH_{-APU-} AADC; CH1_{-APU-}TH_{-other sequence-}AADC; CH1_{-other sequence-}TH-_{APU}-AADC;
CH1_{-APU}-AADC_{-APU-}TH; CH1_{-APU-}AADC_{-other sequence-}TH; CH1_{-other sequence-}AADC_{-APU-}TH;
AADC _{-APU-}TH-_{APU-}CH1; AADC_{-APU-}TH_{-other sequence-}CH1; AADC_{-other sequence-}TH_{-APU}-CH1;
AADC_{-APU-}CH1_{-APU-}TH; AADC_{-APU-}CH1_{-other sequence-}TH; AADC_{-other sequence-}CH1_{-APU-}TH.

APU: auto-processing unit; other sequence: comprising linker peptide coding sequence (linker), internal ribosome entry site (IRES), promoter or intein.

Said auto-processing unit (APU) in the construct comprises a 2A peptide or a 2A-like peptide.

The 2A peptide or 2A-like peptide in the gene sequence construct comprises a 2A peptide derived from foot-and-mouth disease virus (F2A), a 2A peptide derived from porcine teschovirus virus (P2A), a 2A peptide derived from insect virus (T2A), and a 2A peptide derived from equine rhinitis virus (E2A).

If the gene sequence construct comprises a promoter, the promoter is a constitutive promoter or a tissue-specific promoter; a constitutive promoter, comprising CMV promoter, phosphoglycerate kinase promoter, or thymidine kinase promoter. The tissue-specific promoter comprises synapsin promoter, CD68 promoter, GFAP promoter or other synthetic promoters.

Compared with the existing technology, the present invention has the following advantages:
1. The present invention provides a new method for linking genes for dopamine synthesis. The experimental study in the present invention shows that the method can improve the balance of target protein expression, increase the synthesis level of dopamine and its metabolites, and can improve the therapeutic effect against Parkinson's disease.
2. The present invention determines for the first time that the expression of each protein using P2A auto-processing peptide to link the expression of each protein can eventually lead to stable and balanced expression of independently functional tyrosine hydroxylase (TH), GTP-cyclohydrolase I (GCH1), aromatic amino acid dopa decarboxylase (AADC), and a nervous system growth factor, which can provide a new strategy for the treatment and/or prevention of Parkinson's disease and other neurodegenerative diseases.

### DESCRIPTION OF DRAWINGS

In order to more clearly explain the technical solutions of the examples of the present invention or in the existing technology, provided below is a brief introduction of the drawings used to describe the examples or the existing technology. Apparently, the drawings described below are only certain examples of the present invention. For those of ordinary skill in the art, they may also obtain other drawings based on these drawings without creative work.
Figure 1 is a schematic diagram of a gene sequence construct for gene therapy of Parkinson's disease of the present invention.
Figure 2 is a schematic diagram of the structures of the constructs of the present invention.
Figure 3. Western blot detection results for aromatic amino acid dopa decarboxylase (AADC), GTP-cyclohydrolase I (GCH1), and tyrosine hydroxylase (TH) proteins after transduction of 293T cells.
Figure 4. Western blot detection of aromatic amino acid dopa decarboxylase (AADC), GTP-cyclohydrolase I (GCH1), and tyrosine hydroxylase (TH) proteins after transduction of SH-SY5Y cells.
Figure 5. HPLC detection results of dopamine DA production after viral transduction of SH-SY5Y cells.

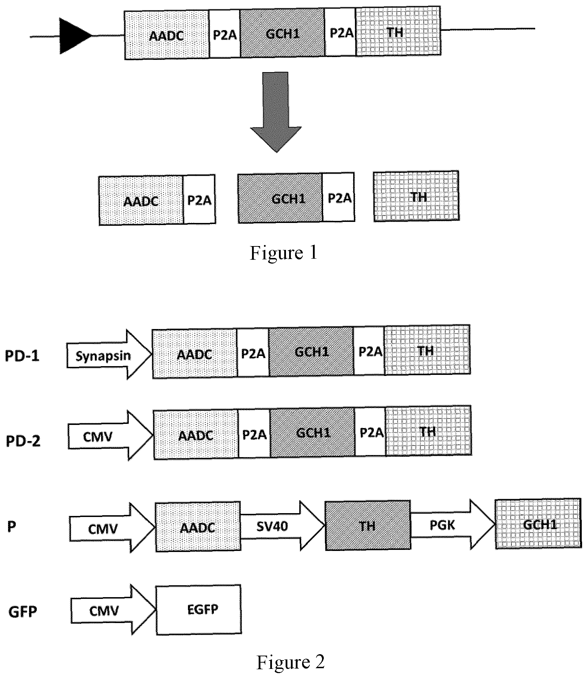

Among them in Figure 1, the coding sequences for the expression of aromatic amino acid dopa decarboxylase (AADC), GTP-cyclohydrolase I (GCH1), and tyrosine hydroxylase (TH) are included. The proteins are linked by auto-processing peptide (P2A). The genes are transcribed, translated, and can produce independent aromatic amino acid dopa decarboxylase (AADC-P2A), GTP-cyclohydrolase I (GCH1-P2A), and tyrosine hydroxylase (TH), after being processed by auto-processing peptide. P2A: porcine teschovirus virus 2A auto-processed peptide.

In Figure 2: AADC: aromatic amino acid dopa decarboxylase; GCH1: GTP-cyclohydrolase I; TH: tyrosine hydroxylase; P2A: porcine teschovirus virus 2A auto-processed peptide; Synapsin, CMV, SV40 and PGK are all promoters.

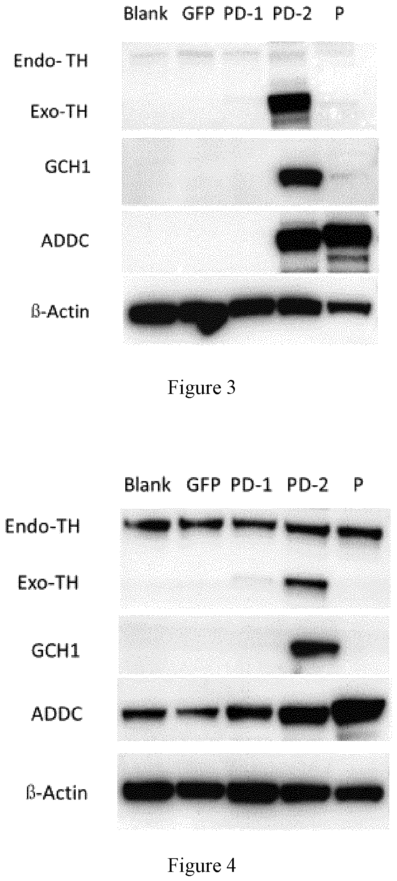

In Figure 3: Blank: cells without viral transduction; GFP: cells transduced with CMV promoter-EGFP virus; PD-1: cells transduced with Synapsin promoter-AADC-P2A-GCH1-P2A-TH virus; PD-2: cells transduced with CMV promoter -AADC-P2A-GCH1-P2A-TH virus; P: cells transduced with CMV promoter-AADC-SV40 promoter-TH-PGK promoter-GCH1 virus; Endo-TH: endogenous TH of the cells; Exo-TH: exogenous overexpressed catalytic domain of TH.

In Figure 4: Blank: cells without viral transduction; GFP: cells transduced with CMV promoter-EGFP virus; PD-1: cells transduced with Synapsin promoter-AADC-P2A-GCH1-P2A-TH virus; PD-2: cells transduced with CMV promoter -AADC-P2A-GCH1-P2A-TH virus; P: cells transduced with CMV promoter-AADC-SV40 promoter-TH-PGK promoter-GCH1 virus; Endo-TH: endogenous TH of the cells; Exo-TH: exogenous overexpressed catalytic domain of TH.

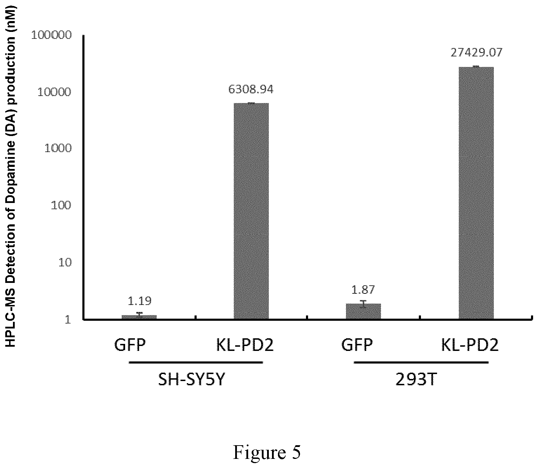

In Figure 5: GFP: cells transduced with GFP virus; PD-2: cells transduced with CMV promoter-AADC-P2A-GCH1-P2A-TH virus.

### DETAILED DESCRIPTION

The present invention is further described in detail below in conjunction with examples. The following examples explain the present invention and the present invention is not limited to the following examples.

### Examples

### I. As shown in Figure 2, the construction of various constructs:

KL0039 vector, synthetic CMV enhancer-synapsin promoter-AADC-P2A-GCH1-P2A-TH and AADC-SV40 promoter-TH-PGK promoter-GCH1 sequences (where TH is a truncated form of TH); wherein, CMV enhancer-synapsin promoter-AADC-P2A-GCH1-P2A-TH is ligated into pUC57 vector (pUC57-synapsin-AGT). Here, the KL0039 vector is a lentiviral transfer vector, derived from existing lentiviral vectors or after partial modifications as needed.
1. Construction of PD1 vector
   A PCR product was amplified from the sequence from WPRE to cPPT using KL0039 as template and primers Age-F and Sal-R and purified after electrophoresis. The primer sequences are: Age-F, CTGAGTGCCATTGGATGAcaatcaacctctggattaca; Sal-R, gattactattaataactactcacgcatgctcttctcca. Plasmid pUC57-synapsin-AGT was digested with AgeI and SalI, and the 4.1-kb fragment was recovered. The ligation products of the purified PCR product and synapsin-AGT fragment by T4 DNA ligase were used to transform DH5α competent cells. Transformant colonies were screened by PCR and the positive clones were further confirmed by sequencing.
2. Construction of PD2 vector
   Using KL0039 vector as template and primers SnaBI-F:
   TCAGtacgtattagtcatcgctat and SpeI-R:
   CGATactagtgagctctgcttatataga, a PCR product (245 bp) of CMV promoter was amplified and purified after electrophoresis. Double digestion by SnaBI and SpeI was performed on the purified PCR product of CMV promoter and plasmid pUC57-synapsin-AGT, respectively, and the fragments of CMV promoter and pUC57-AGT were purified from the digestion products. The ligation product of the two fragments by T4 DNA ligase was used to transform DH5α competent cells. Transformant colonies were screened by PCR and the positive clones were further confirmed by sequencing. The positive clone was named pUC57-CMV-AGT. Then, a PCR product was amplified from the sequence from WPRE to cPPT using KL0039 as template and Age-F+Sal-R and purified after electrophoresis. Double digestion by AgeI and SalI was performed on the purified PCR product and plasmid pUC57-CMV-AGT, respectively, and the fragments of the PCR product and CMV-AGT were purified from the digestion products. The ligation product of the two fragments by T4 DNA ligase was used to transform DH5α competent cells. Transformant colonies were screened by PCR and the positive clones were further confirmed by sequencing.
3. Construction of P vector: the AADC-SV40 promoter-TH-PGK promoter-GCH1 sequence was used to replace the AGT sequence in PD2 vector.
4. GFP vector: the EGFP sequence was cloned and used to replace the AGT sequence in PD2 vector.

### II. Evaluation of the differential expression of target proteins in 293T and SH-SY5Y cells after transduction with various constructs

Lentiviral four-plasmid system was used to transiently transfect 293T cell line, packaging GFP (CMV promoter-EGFP), PD1 (synapsin promoter-AGT), PD2 (CMV promoter-AGT) lentivirus and positive control virus P (CMV promoter-AADC-SV40 promoter-TH-PGK promoter-GCH1), respectively. The initial viruses were concentrated after purification and transduced into 293T cells after dilution. The titers were determined using RT-PCR (WPRE/ALB). The vector titers of all constructs were similar, ranging from 3.4E+09TU/ml to 8.74E+09TU/ml.

In order to assess the expression levels of target proteins, 293T cells and SH-SY5Y cells were transduced with the lentiviruses at MOI=10 and MOI=20, respectively. The cells were harvested 72 hours after transduction and cell lysates were used for Western blot analysis of AADC, GCH1, and TH. The results show that a relatively low level of endogenous TH, but no endogenous AADC and GCH1, was detected in 293T cells. The molecular weights of all target proteins were consistent with the expected values. Compared with no virus transduction Blank and GFP virus transduction, high levels of expression of all three target proteins were detected from cells transduced with PD2 viral vector. Although cells transduced with P viral vector expressed the highest level of AADC, the other two target proteins GCH1 and TH were barely detected. As expected, no expression of the three target proteins was detected in 293T cells transduced with PD1 viral vector, in which the synapsin promoter used is neuron-specific (Figure 3). Further, we assessed the expression of targeted proteins in SH-SY5Y cells after transduction with the three different constructs. Endogenous TH and AADC with expected molecular weights were detected, but not endogenous GCH1. Similar to the results from transduced 293T cells, high levels of expression of all three target proteins were detected from cells transduced with PD2 viral vector, when compared with no virus transduction Blank and GFP virus transduction. Although cells transduced with P viral vector expressed the highest level of AADC, the other two proteins GCH1 and TH were still barely detected. For cells transduced with PD1 viral vector, only low levels of exogenous AADC and TH were expressed, and GCH1 was barely detected (Figure 4).

### III. Evaluation of the differential catecholamine production in SH-SY5Y cells after transduction with various constructs

In neurons, DA is converted primarily by monoamine oxidase (MAO) to dihydroxyphenylacetic acid (DOPAC). The levels of catecholamine were measured by mass spectrometry in the supernatants of two cultured cells, SH SY5Y cells and 293T cells, after transduction with lentiviral vectors. The SH SY5Y cells were transduced by viruses and the media were replaced after overnight. The supernatants were collected after being cultured until the third day and centrifuged at 4500 rpm for 5 minutes. The clear supernatants were transferred to 1.5mL centrifugation tubes and stored in freezer at -80°C before testing. The 293T cells were transduced by viruses and the media were changed after overnight. After 2 days of culture, the cells were passaged at 1:10. After 2 days of culture, the media were replaced with fresh media containing 10 mM L-tyrosine. The supernatants were collected after being cultured until the next morning and centrifuged at 4500 rpm for 5min. The clear supernatants were transferred to 1.5mL centrifuge tubes and stored in freezer at -80°C before testing.

The levels of dopamine in the samples were measured by mass spectrometry as follows. 500 µL of cell culture medium was collected and an appropriate amount of internal standard was added. The solution was diluted and mixed with 1 mL of 50 mM ammonium acetate serving as the sample loading solution. After methanol activation, the cartridge was subsequently rinsed with 20 mM ammonium acetate, acetonitrile: isopropanol (1:1), and drained. The sample was eluted with 2% formic acid in acetonitrile and blown dry with nitrogen. The residue was dissolved in 100 µL of 0.1% FA and centrifuged at 15000 r/min for 5 min. The supernatant was loaded onto the machine (Angilent 1290UPLC-6470MS/MS detection system) for analysis.

The results are shown in Figure 5. Transduction of 293T cells and SH-SY5Y cells with KL-PD2 lentiviral vector greatly increased the production of dopamine in the cell culture supernatants due to the effective and balanced expression of the three enzymes in dopamine synthesis.

In addition, it should be noted that the specific examples described in this specification may bear different names for various substances or carriers. Any equivalent or simple variation made according to the structure configuration and principles described in the patent conception of the present invention all belong to the scope of the present patent protection. Those skilled in the art to which the present invention pertains can make various modifications or additions to the described specific examples or substitute in a similar manner, as long as they do not depart from the structures of the present invention or go beyond the scope defined by the claims, all should belong to the scope of protection of the present invention.

## Claims

1. A gene sequence construct for the treatment of a central nervous system disease, **characterized in that** the construct comprises nucleotide sequences that are related to the treatment of the central nervous system disease and are linked by an auto-processing unit (APU).

2. The gene sequence construct of claim 1 for the treatment of a central nervous system disease, **characterized in that** said auto-processing unit (APU) comprises an N-terminal auto-processing domain and/or a C-terminal auto-processing domain.

3. The gene sequence construct of claim 2 for the for the treatment of a central nervous system disease, **characterized in that** said N-terminal auto-processing domain comprises Intein, B-type bacterial intein-like domain (BIL), Furin sequence, or a derivative thereof;
said C-terminal auto-processing domain comprises a 2A peptide or a 2A-like peptide.

4. The gene sequence construct of claim 3 for the for the treatment of a central nervous system disease, **characterized in that**, the 2A peptide or 2A-like peptide comprises a 2A peptide derived from foot-and-mouth disease virus (F2A), a 2A peptide derived from porcine teschovirus virus (P2A), a 2A peptide derived from insect virus (T2A), or a 2A peptide derived from equine rhinitis virus (E2A).

5. The gene sequence construct of claim 1 for the treatment of a central nervous system disease, **characterized in that** said nucleotide sequences related to the central nervous system disease comprise selection from two or more of the nucleotide sequences of tyrosine hydroxylase (TH), GTP-cyclohydrolase I (GCH1), aromatic amino acid dopa decarboxylase (AADC), or a nervous system growth factor;
said nucleotide sequences comprising at least two nucleotide sequences that are linked by an auto-processing unit (APU).

6. The gene sequence construct of claim 5 for the treatment of a central nervous system disease, **characterized in that** the nervous system growth factor comprises nerve growth factor (NGF), brain-derived neurotrophic factor (BDNF), neurotrophin-3 (NT-3), neurotrophin-4/5 (NT-4/5), neurotrophin-6 (NT-6), ciliary neurotrophic factor (CNTF), glial cell line-derived neurotrophic factor (GDNF) or a GDNF family molecule;
said GDNF family molecule comprises a naturally occurring analog of GDNF, neurturin, persephin or artemin.

7. The gene sequence construct of claim 5 for the treatment of a central nervous system disease, **characterized in that** the construct comprises tyrosine hydroxylase (TH), GTP-cyclohydrolase I (GCH1), aromatic amino acid dopa decarboxylase (AADC);
said nucleotides sequences comprising at least two nucleotide sequences that are linked by an auto-processing unit (APU).

8. A viral vector genome, **characterized in that** said viral vector genome comprises a gene sequence construct of any one of claims 1-7 for the treatment of a central nervous system disease, said viral vector comprises a lentiviral vector or an adeno-associated viral vector.

9. A lentiviral vector system, **characterized in that** said lentiviral vector system comprises a genome comprising the gene sequence construct of any one of claims 1-7 for the treatment of a central nervous system disease.

10. A biological product comprising a gene sequence construct of any one of claims 1-7, and its use in the treatment and/or prevention of Parkinson's disease, Alzheimer's disease and other neurodegenerative diseases.
